# EUROPÄISCHE PATENTANMELDUNG

(11) **EP 4 338 778 A1**
(43) Veröffentlichungstag der Anmeldung: **20.03.2024**
(21) Anmeldenummer: 22195290.6
(22) Anmeldetag: 13.09.2022
(51) Int. Cl.: A61M 25/00, A61B 5/00, A61B 1/00

(54) **WIEDERVERWENDBARE KOMMUNIKATIONSEINRICHTUNG FÜR EINEN KATHETER ZUR LÖSBAREN VERBINDUNG MIT EINEM KATHETERHUB DES KATHETERS**

(71) Anmelder: BIOTRONIK AG, 8180 Bülach (CH)
(72) Erfinder: Quint, Bodo, 79802 Dettighofen (CH)
(74) Vertreter: Biotronik Corporate Services SE

(57) **Zusammenfassung**

Die Anmeldung betrifft eine Kommunikationseinrichtung (1) zur Nahfeldkommunikation mit einem Katheter (100), mit: einer NFC-Spule (3) und einem damit verbundenen Kommunikationsschaltkreis (4), der für eine bidirektionale Nahfeldkommunikation mit dem Katheter (100) konfiguriert ist, einem wiederaufladbaren Energiespeicher (5), einer elektrischen Ladespule (6), die mit einem Ladeschaltkreis (7) zum Aufladen des Energiespeichers (5) verbunden ist, und einem Gehäuse (2), das die NFC-Spule (3), den Kommunikationsschaltkreis (4), den Energiespeicher (5), die Ladespule (6) und den Ladeschaltkreis (7) umgibt, wobei das Gehäuse (2) dazu ausgebildet ist, zur Nahfeldkommunikation (N1) mit dem Katheter (100) lösbar an dem Katheter (100) festgelegt zu werden.

## Beschreibung

Die Erfindung betrifft eine Kommunikationseinrichtung für einen Katheter zur lösbaren mechanischen Verbindung mit einem Katheterhub des Katheters. Weiterhin betrifft die Erfindung ein medizinisches System mit einer solchen Kommunikationseinrichtung.

Bei dem Katheter kann es sich z.B. um einen Ballonkatheter handeln, der im Rahmen einer Angioplastie dazu verwendet wird, eine Stenose mittels eines Stents aufzuweiten, der auf einem Ballon des Ballonkatheters angeordnet ist und mittels des Ballonkatheters zum Zielort transportiert wird und dort mittels des Ballons aufgeweitet wird bzw. im Gefäß zur Beseitigung der Engstelle verankert wird. Der Katheter kann dabei z.B. einen Sensor zur Bestimmung des Ballondurchmessers aufweisen. Andere Sensoren sind ebenfalls denkbar. Alternativ kann der Katheter z. B. zur Freisetzung eines Medikaments im Patienten vorgesehen sein.

Hierzu notwendige elektrische Funktionalitäten des jeweiligen Katheters bedeuten jedoch, dass eine geeignete Stromversorgung und Signal- oder Datenkonnektivität zu dem jeweiligen Katheter realisiert werden muss.

Eine direkte Verbindungstechnik zu dem Katheter birgt allerdings das Risiko einer Einschränkung des taktilen Handlings des Benutzers sowie Nachteile hinsichtlich der Sterilisierbarkeit und Haltbarkeit der elektrischen Verbindungen.

Hiervon ausgehend liegt der vorliegenden Erfindung daher die Aufgabe zugrunde, einen Katheter dahingehend zu modifizieren, dass ein einfacher Datenaustausch des Katheters mit weiteren Komponenten eines medizinischen Systems gewährleistet ist und eine effiziente Sterilisierbarkeit des Katheters und damit verbundener bzw. verbindbarer Funktionskomponenten möglich ist.

Diese Aufgabe wird durch eine Kommunikationseinrichtung mit den Merkmalen des Anspruchs 1 sowie durch ein medizinisches System mit den Merkmalen des Anspruchs 8 gelöst. Vorteilhafte Ausgestaltungen dieser Erfindungsaspekte sind in den entsprechenden Unteransprüchen angegeben und werden nachfolgend beschrieben.

Gemäß Anspruch 1 wird eine Kommunikationseinrichtung zur Nahfeldkommunikation mit einem Katheter offenbart, wobei die Kommunikationseinrichtung aufweist:
- eine elektrische NFC-Spule und einem damit verbundenen Kommunikationsschaltkreis, der für eine bidirektionale Nahfeldkommunikation mit dem Katheter (insbesondere mit einem NFC-Transponder des Katheters) konfiguriert ist,
- einen wiederaufladbaren Energiespeicher,
- eine elektrischen Ladespule, die mit einem Ladeschaltkreis zum Aufladen des Energiespeichers verbunden ist, und
- ein Gehäuse, das die NFC-Spule, den Kommunikationsschaltkreis, den Energiespeicher, die Ladespule und den Ladeschaltkreis umschließt, wobei das Gehäuse dazu ausgebildet ist, zur Nahfeldkommunikation mit dem Katheter lösbar an dem Katheter festgelegt zu werden.

Besonders bevorzugt ist gemäß einer Ausführungsform der Erfindung vorgesehen, dass die Kommunikationseinrichtung bzw. das Gehäuse lösbar an einem Katheterhub des Katheters festlegbar ist. Der Katheterhub ist an einem proximalen Ende des Katheters vorgesehen und weist insbesondere einen Anschluss, z.B. einen Luer-Konnektor, zum Anschließen einer Leitung auf, über die ein fluides Medium in ein Lumen des Katheters einleitbar ist.

Der Begriff Nahfeldkommunikation (kurz NFC für Near Field Communication) bezeichnet einerseits den gleichnamigen Standard (NFC) sowie andererseits das zugrundeliegende technische Prinzip eines kontaktlosen Datenaustausches bei einer Betriebsfrequenz von 13,56 MHz.

Die Nahfeldkommunikation beruht in der Regel auf einer Kommunikation zwischen einem Lesegerät (hier aufweisend die NFC-Spule sowie den Kommunikationsschaltkreis der Kommunikationseinrichtung) und einem passiven NFC-Transponder, hier vorzugsweise im Katheter angeordnet, der seine Versorgungsspannung aus dem vom Lesegerät bzw. der NFC-Spule erzeugten Magnetfeld bezieht. Der Transponder weist ebenfalls eine Leiterschleife bzw. NFC-Spule zur Wechselwirkung bzw. induktiven Kopplung mit der NFC-Spule der Kommunikationseinrichtung auf.

Über die induktive Kopplung von Lesegerät und Transponder wird zum einen die Energie zur Versorgung des Transponders übertragen, zum anderen müssen aber auch Daten zwischen dem Lesegerät und dem Transponder ausgetauscht werden. Diese Datenübertragung ist z.B. in ISO/IEC 14443-2 für RFID-Systeme bei 13,56 MHz festgehalten. In der Regel kommunizieren Lesegerät und Transponder nach dem RTF-Prinzip (RTF für Reader Talks First). Dabei wird eine Kommunikation bzw. Datenübertragung vom Lesegerät initiiert, der Transponder sendet Daten lediglich als Antwort auf ein entsprechendes Signal des Lesegerätes. Die Daten- bzw. Signalübertragung vom Lesegerät zum Transponder erfolgt über eine direkte Amplitudenmodulation des Trägersignals. Demgegenüber erfolgt die Daten- bzw. Signalübertragung vom Transponder zum Lesegerät mittels Lastmodulation mit Hilfsträger. Lastmodulation bedeutet diesbezüglich, dass der Transponder seine Impedanz variiert, um die Übertragungsenergie mit dem Datenstrom zu modulieren. Durch die induktive Kopplung der NFC-Spule (Lesegerät) der Kommunikationseinrichtung mit der NFC-Spule des Transponder des Katheters, bewirkt diese Laständerung Strom- und Spannungsänderungen in der NFC-Spule der Kommunikationseinrichtung, die über den besagten Kommunikationsschaltkreis detektiert werden können.

Die erfindungsgemäße Kommunikationseinrichtung ermöglicht vorteilhafte funktionelle Eigenschaften, wie z.B. eine einfache und vollständige Sterilisierbarkeit des Systems sowie gleichzeitig ein unterstützendes Katheterhandling, insbesondere durch eine ergonomische Formgebung des Gehäuses der Kommunikationseinrichtung.

Weiterhin kann die Erfindung universell auf aller erdenklichen Kathetersysteme angewendet werden und erlaubt in diesem Zusammenhang die Umsetzung von elektrischen Katheter-Funktionen (z.B. Ballondilatation, SDS, Drainagekatheter, Drainagekatheter mit Medikamentierungsfunktion, Drainagekatheter mit zeitlich gesteuerter Funktionalität, Führungskatheter mit zusätzlicher Sensorik) da mittels der erfindungsgemäßen Kommunikationseinrichtung direkt die Energieübertragung zu dem Katheter möglich ist und dessen Funktionen über die Nahfeldkommunikation steuerbar sind. Weiterhin erlaubt die Erfindung somit eine Kostenreduktion hinsichtlich des jeweiligen Katheters.

Ferner kann die erfindungsgemäße Kommunikationseinrichtung an Verbindungssystemen für Infusions- und Injektionsvorrichtungen, insbesondere an einem Luer-System, oder auch an mit solchen Verbindungssystemen verbindbaren Vorrichtungen, beispielsweise einem mit einen Luer-Konnektor versehenden Durchflussventil, lösbar mechanisch verbunden werden und eine kommunikative Kopplung zwischen dem Lesegerät der Kommunikationseinrichtung und einem Transponder des Verbindungssystems bzw. der mit einem solchen Verbindungssystem verbindbaren Vorrichtung zur Daten- und Signalübertragung erzielt werden. Auf diese Weise kann die Kommunikationseinrichtung beispielsweise verwendet werden, um eine mit dem Verbindungssystem verbundene Infusions- oder Inj ektionsvorrichtungen zu steuern, beispielsweise für die Medikamentenabgabe über ein steuerbares Ventil einer Infusionsvorrichtung.

Die besagte NFC-Technologie kann besonders vorteilhaft im Zusammenhang mit der vorliegenden Erfindung verwendet werden, da hier eine Energieübertragung bzw. Kommunikation in einem Nahfeldbereich (einige mm bis wenige cm) über entsprechende NFC-Spulen bzw. -Antennen stattfindet, deren Reichweite wegen der Nähe zwischen der Kommunikationseinrichtung und dem Katheter vollkommen ausreichend ist und gleichzeitig einen verhältnismäßig niedrigen Energieverbrauch aufweist.

Die Datenübertragung beruht auf einer induktiven Kopplung (siehe oben), die in der Regel auf eine Übertragungsfrequenz von 13.56MHz ausgelegt ist und von den notwendigen Komponenten her vorteilhaft kleinbauend ausfällt und daher den Dimensionen entspricht, die durch einen typisches Katheter-Luer bzw. einen typischen Katheterhub realisiert sind.

Der NFC-Kommunikationsschaltkreis der Kommunikationseinrichtung kann mit Vorteil programmierbar ausgestaltet sein, nicht ausschließlich zur Speicherung von Dateninhalten, so dass auch komplexe funktionale Erweiterungen realisierbar sind. Z. B. können ARM-Prozessorkerne mit frei programmierbaren Ressourcen in solche Kommunikationsbausteine integriert werden, so dass auch entsprechende Rechenleistung verfügbar ist.

Bevorzugt ist gemäß einer Ausführungsform der Erfindung vorgesehen, dass das Gehäuse dazu ausgestaltet ist, mittels einer Klemmverbindung mit dem Katheter bzw. mit dem Katheterhub verbunden zu werden. Das Gehäuse kann hierzu Rastzungen aufweisen, die jeweils dazu konfiguriert sein können, einen Materialbereich des Katheters bzw. des Katheterhub zur Ausbildung der lösbaren Verbindung zu hintergreifen.

Gemäß einer bevorzugten Ausgestaltung der Kommunikationseinrichtung ist vorgesehen, dass das Gehäuse als Griff ausgestaltet ist.

Weiterhin ist gemäß einer Ausführungsform der Kommunikationseinrichtung vorgesehen, dass das Gehäuse dazu ausgestaltet ist, zum Handhaben des Katheters einhändig gegriffen zu werden, wenn das Gehäuse lösbar mit dem Katheterhub des Katheters verbunden ist.

Weiterhin ist gemäß einer Ausführungsform der Kommunikationseinrichtung vorgesehen, dass das Gehäuse eine Mulde zur Aufnahme eines Daumens eines Verwenders (insbesondere eines Arztes) der Kommunikationseinrichtung aufweist, und/oder dass das Gehäuse eine Mulde für einen Zeigefinger eines Verwenders (insbesondere eines Arztes) aufweist.

Weiterhin kann das Gehäuse gemäß einer Ausführungsform eine Ausnehmung zur Aufnahme eines Bereichs des Katheters, insbesondere des Katheterhubs, aufweisen.

Weiterhin ist gemäß einer Ausführungsform der Kommunikationseinrichtung vorgesehen, dass das Gehäuse die besagten Komponenten (die NFC-Spule, den Kommunikationsschaltkreis, den Energiespeicher, die Ladespule und den Ladeschaltkreis sowie ggf. weitere Komponenten) hermetisch umschließt und insbesondere über keinerlei Fügestellen oder Öffnungen verfügt. Das Gehäuse kann z.B. aus einem Kunststoff wie z. B. POM (Polyoxymethylen) hergestellt sein. Das Gehäuse kann aus einem Harz gebildet sein, in das die besagten Komponenten eingegossen sind. Weiterhin kann das Gehäuse durch Umspritzen der besagten Komponenten mit dem Kunststoff gebildet sein.

Weiterhin ist gemäß einer Ausführungsform der Kommunikationseinrichtung vorgesehen, dass die Kommunikationseinrichtung eine Sende-Empfangseinheit (d.h. einen Transceiver) aufweist, die zur Kommunikation mit einer Andockstation konfiguriert ist. Die Kommunikation kann z.B. bei einer Übertragungsfrequenz von 433Mhz / 898 MHz erfolgen.

Weiterhin kann diese Kommunikation nach einem WLAN-Standard (z.B. aus der IEEE-802.11-Familie), einem WPAN bzw. Bluetooth-Standard oder z.B. nach MICS/MEDS 400 MHz Übertragungsstandard (fda/eu) erfolgen.

Gemäß einer Ausführungsform der Erfindung ist die Ladespule der Kommunikationseinrichtung eine NFC-Ladespule zum Aufladen des Energiespeichers an der Andockstation, wobei die NFC-Ladespule dazu konfiguriert ist, auch die Kommunikation für eine Überwachung und/oder einen Systemtest und/oder eine Konfiguration der Kommunikationseinrichtung zu übernehmen. Die Ladung des Energiespeichers sowie die besagte Konfiguration erfolgen also ebenfalls vorzugsweise über eine induktive Kopplung im Rahmen einer Nahfeldkommunikation (NFC).

Weiterhin ist gemäß einer Ausführungsform der Kommunikationseinrichtung vorgesehen, dass die Kommunikationseinrichtung eine optische Anzeige aufweist, insbesondere in Form zumindest einer Leuchtdiode (LED), wobei die Kommunikationseinrichtung dazu konfiguriert ist, einen Zustand der Kommunikationseinrichtung mittels der optischen Anzeigeeinrichtung anzuzeigen.

Bei dem Zustand kann es sich z.B. um einen Ladezustand des Energiespeichers handeln oder um einen momentan erfolgenden Ladevorgang des Energiespeichers. Weiterhin kann es sich bei dem Zustand um eine momentan erfolgende Kommunikation zwischen der Kommunikationseinrichtung und dem Katheter und/oder zwischen der Kommunikationseinrichtung und einer Andockstation für die Kommunikationseinrichtung.

Vorzugsweise ist die optische Anzeige vollständig vom Gehäuse der Kommunikationseinrichtung umgeben, d.h., das Gehäuse weist keine Durchgangsöffnung in einer Gehäusewandung des Gehäuses für die optische Anzeige aus. Das Gehäuse kann für die optische Anzeige einen die optische Anzeige überdeckenden transluzenten oder transparenten Bereich aufweisen.

Gemäß einer Ausführungsform der Erfindung kann der Katheter ein Drainagekatheter ein, wobei die Kommunikationseinrichtung dazu ausgebildet ist, den Katheter über die NFC-Spule der Kommunikationseinheit zur Abgabe eines Medikaments an einen Patienten zu veranlassen.

Auf diese Weise kann mittels der Kommunikationseinrichtung eine Medikamentenabgabe initiiert und zeitgesteuert erfolgen. Die Funktion hierzu kann während eines Sterilisations- und/oder Ladevorganges konfiguriert werden, der erfolgt, wenn die Kommunikationseinheit an die Andockstation angedockt ist. Die Kommunikationseinrichtung kann hierbei ein Signal an die Andockstation übermitteln, das anzeigt, dass die Abgabe des Medikaments korrekt vorgenommen wird bzw. vorgenommen wurde.

Weiterhin ist gemäß einer Ausführungsform der Kommunikationseinrichtung vorgesehen, dass der Katheter (z.B. kann es sich bei dem Katheter um einen Ballonkatheter handeln) zumindest einen Sensor aufweist, insbesondere einen resistiven oder kapazitiven Sensor, z.B. einen Sensor zur Durchmesserbestimmung eines Ballons des Ballonkatheters, wobei die Kommunikationseinrichtung dazu ausgebildet ist, ein Ausgangssignal des mindestens einen Sensors und/oder daraus ermittelte Daten mittels der NFC-Spule und dem Kommunikationsschaltkreis auszulesen und vorzugsweise an eine Andockstation weiterzugeben.

Auf diese Weise kann die Kommunikationseinrichtung die mittels des Katheters gewonnenen Messwerte an ein übergeordnetes Analysesystem (z.B. über die Andockstation) weitergeben.

Weiterhin ist gemäß einer Ausführungsform der Kommunikationseinrichtung vorgesehen, dass die Kommunikationseinrichtung dazu konfiguriert ist, über die NFC-Spule katheterspezifische Daten, insbesondere der Durchmesser des Ballons und/oder Kalibrierungsdaten eines Sensors, beispielsweise eines Drucksensors, aus dem Katheter auszulesen.

Diesbezüglich kann die Kommunikationseinrichtung dazu ausgebildet sein, die ausgelesenen katheterspezifischen Daten an ein übergeordnetes System weiterzugeben (z.B. über die Andockstation), so dass dort die besagten Daten detailliert visualisierbar sind. So können z.B. sämtliche für einen Kathetereingriff notwendigen Informationen auf einem Bildschirm des übergeordneten Systems dargestellt werden. Die spezifisch erfassten Daten können so sowohl die Identifikation / Klassifikation des Katheters wie auch eine zusätzliche Detailierung von Kathetereigenschaften durch z.B. katheterspezifische Kalibrierungsdaten erlauben. Hierbei können weiterhin (z.B. mittels erfassender Sensorik am Katheter) noch zusätzliche Eigenschaften und/oder Instruktionen und/oder Handlungsempfehlungen und/oder Messergebnisse an den Benutzer des Katheters weitergegeben werden.

Ein weiterer Aspekt der Erfindung betrifft ein medizinisches System aufweisend zumindest eine (oder mehrere) erfindungsgemäße Kommunikationseinrichtung(en) sowie eine separate Andockstation, wobei die mindestens eine Kommunikationseinrichtung an die Andockstation andockbar ist.

Gemäß einer Ausführungsform der Erfindung kann die Andockstation ein Eingabemittel, z.B. in Form einer Tastatur, zum Eingeben von Information bzw. zum Programmieren der Andockstation bzw. der mindestens einen Kommunikationseinrichtung aufweisen.

Weiterhin ist gemäß einer Ausführungsform des medizinischen Systems vorgesehen, dass die Andockstation zum Andocken der mindestens einen Kommunikationseinrichtung eine Aufnahme zum Aufnehmen der mindestens einen Kommunikationseinrichtung aufweist. Angedockt sein bedeutet dabei insbesondere, dass die mindestens eine Kommunikationseinrichtung in einer definierten Position bezüglich der Andockstation angeordnet ist, die ein Laden des Energiespeichers der Kommunikationseinrichtung mittels der Andockstation gestattet. Dies kann z.B. durch ein Anordnen der Kommunikationseinrichtung in der zugeordneten Aufnahme der Andockstation sichergestellt werden.

Weiterhin ist gemäß einer Ausführungsform der Erfindung vorgesehen, dass die Andockstation dazu ausgebildet ist, die mindestens eine Kommunikationseinrichtung mit einer Lösung zu Spülen insbesondere zu Reinigungs- und/oder Pflegezwecken und/oder die mindestens eine Kommunikationseinrichtung durch Kontaktieren mit einem Sterilisationsmittel zu sterilisieren, wenn die mindestens eine Kommunikationseinrichtung an die Andockstation angedockt ist. Das Sterilisationsmittel kann z.B. als sterilisierende Flüssigkeit vorliegen, die in die Aufnahme für die Kommunikationseinrichtung eingeleitet wird.

Weiterhin ist gemäß einer Ausführungsform des medizinischen Systems vorgesehen, dass die Andockstation zum Laden des Energiespeichers der mindestens einen Kommunikationseinrichtung über die Ladespule ausgebildet ist, wenn die mindestens eine Kommunikationseinrichtung an die Andockstation angedockt ist (also z.B. in der zugeordneten Aufnahme der Andockstation angeordnet ist), und/oder dass die Andockstation zur Durchführung eines Systemtests der mindestens einen Kommunikationseinrichtung über die Ladespule ausgebildet ist, wenn die mindestens eine Kommunikationseinrichtung an die Andockstation angedockt ist (also z.B. in der zugeordneten Aufnahme der Andockstation angeordnet ist).

Die Andockstation kann insbesondere Überwachungsfunktionalitäten aufweisen. So kann die Andockstation gemäß einer Ausführungsform dazu ausgebildet sein, einen Ladezustand des Energiespeichers der mindesten einen Kommunikationseinrichtung anzuzeigen. Der Ladezustand beschreibt dabei die aktuelle Kapazität des Energiespeichers im Verhältnis zur maximalen Kapazität des Energiespeichers. Weiterhin kann die Andockstation gemäß einer Ausführungsform dazu ausgebildet sein, ein Warnsignal auszugeben, z.B. wenn der Ladezustand unter einen vordefinierten Schwellenwert sinkt.

Weiterhin kann die Andockstation gemäß einer Ausführungsform des medizinischen Systems dazu ausgebildet sein, eine erfolgte Gerätepaarung zwischen der mindesten einen Kommunikationseinrichtung und dem Katheter anzuzeigen.

Weiterhin ist gemäß einer Ausführungsform des medizinischen Systems vorgesehen, dass die Andockstation dazu ausgebildet ist, die mindestens eine Kommunikationseinrichtung über die Ladespule zu konfigurieren, so dass insbesondere die mindestens eine Kommunikationseinrichtung nach dem Konfigurieren eine Funktion des Katheters über die NFC-Spule der mindestens einen Kommunikationseinrichtung steuern kann und/oder Daten aus dem Katheter auslesen kann.

Die Konfigurationseinstellungen können weiterhin z.B. die Einstellung eines Funkkanals zwischen der Andockstation und der Kommunikationseinrichtung bzw. zwischen dem Katheter und der Kommunikationseinrichtung betreffen sowie eine ID der jeweiligen Kommunikation.

Weiterhin ist gemäß einer Ausführungsform des medizinischen Systems vorgesehen, dass die Andockstation eine Sende-Empfangseinheit (d.h. einen Transceiver) aufweist, die zur Kommunikation mit der Sende-Empfangseinheit der mindestens einen Kommunikationseinrichtung konfiguriert ist, insbesondere wenn die mindestens eine Kommunikationseinrichtung bestimmungsgemäß lösbar an dem Katheterhub des Katheters festgelegt ist.

Weiterhin ist gemäß einer Ausführungsform des medizinischen Systems vorgesehen, dass das medizinische System zumindest einen Rechner aufweist, wobei die Andockstation dazu ausgebildet ist, über eine Datenaustauschverbindung an den mindestens einen Rechner Daten zu übermitteln, die durch die mindestens eine Kommunikationseinrichtung an die Andockstation übermittelt worden sind. Der mindestens eine Rechner kann zur Weiterverarbeitung und/oder Visualisierung der übermittelten Daten konfiguriert sein.

Weiterhin kann der mindestens eine Rechner Teil eines Rechnernetzwerks sein, insbesondere ein Server des Rechnernetzwerks. Bei dem Rechnernetzwerk kann es sich um ein Rechnernetzwerk eines Home Monitoring Service Centers (HMSC) handeln, wobei das Rechnernetzwerk dazu ausgebildet ist Patientendaten zu verwalten bzw. zu überwachen. Diesbezüglich kann das Rechnernetzwerk oder der mindestens eine Rechner dazu konfiguriert sein, eine Verknüpfung von katheterspezifischen Daten mit Daten eines Eingriffes (unter Verwendung des Katheters) sowie mit Patientendaten des behandelten Patienten herzustellen, insbesondere zur Erzeugung einer Handlungsempfehlung hinsichtlich eines zukünftigen Eingriffs an dem gleichen Patienten oder einem anderen Patienten. Die Datenaustauschverbindung kann einen oder mehrere Verbindungstypen verwenden (z.B. Ethernet, WLAN, ein Mobilfunknetzwerk, eine Netzwerkverbindung gemäß Internetprotokoll etc.)

Gemäß einer Ausführungsform des medizinischen Systems ist die mindestens eine Kommunikationseinrichtung dazu ausgebildet, eine Medikamentenfreigabe des Katheters zu initiieren und zu steuern, wobei die Andockstation dazu ausgebildet ist, eine entsprechende Konfiguration der mindestens einen Kommunikationseinrichtung an die mindestens eine Kommunikationseinrichtung zu übertragen bzw. vorzunehmen, wenn die mindestens eine Kommunikationseinrichtung an die Andockstation angedockt ist, wobei die Konfiguration z.B. während eine Sterilisationsvorgangs und/oder eines Ladevorganges des Energiespeichers der mindestens einen Kommunikationseinrichtung vorgenommen wird.

Weiterhin ist gemäß einer Ausführungsform des medizinischen Systems vorgesehen, dass die mindestens eine Kommunikationseinrichtung dazu ausgebildet ist, ein Ausgangssignal des mindestens einen Sensors und/oder daraus ermittelte Daten mittels der NFC-Spule und des Kommunikationsschaltkreises der mindestens einen Kommunikationseinrichtung auszulesen und an die Andockstation weiterzugeben, wobei die Andockstation dazu ausgebildet ist, das Ausgangssignal und/oder die besagten Daten über die Datenaustauschverbindung an den mindestens einen Rechner des medizinischen Systems zu übermitteln.

Weiterhin ist gemäß einer Ausführungsform des medizinischen Systems vorgesehen, dass die mindestens eine Kommunikationseinrichtung dazu ausgebildet ist, mittels der NFC-Spule und des Kommunikationsschaltkreises der mindestens einen Kommunikationseinrichtung katheterspezifische Daten aus dem Katheter auszulesen und an die Andockstation zu übermitteln, wobei die Andockstation dazu ausgebildet ist, die katheterspezifischen Daten über die Datenaustauschverbindung an den mindestens einen Rechner zu übermitteln, der insbesondere dazu ausgebildet ist, die katheterspezifischen Daten und/oder daraus erzeugte Daten auf einem Bildschirm darzustellen oder anderweitig zu verarbeiten.

Der mindestens eine Rechner kann insbesondere detailliert sämtliche für einen Kathetereingriff notwendigen Informationen auf dem Bildschirm darstellen. Die spezifisch erfassten Daten erlauben insbesondere sowohl die Identifikation / Klassifikation des Katheters wie auch eine zusätzliche Detailierung von Kathetereigenschaften durch z.B. katheterspezifische Kalibrierungsdaten.

Weiterhin ist gemäß einer Ausführungsform des medizinischen Systems vorgesehen, dass die mindestens eine Kommunikationseinrichtung dazu ausgebildet ist, mittels der NFC-Spule und des Kommunikationsschaltkreises der Kommunikationseinrichtung Sensordaten eines oder mehrerer Sensoren des Katheters aus dem Katheter auszulesen und an die Andockstation zu übermitteln, wobei die Andockstation dazu ausgebildet ist, die Sensordaten über die Datenaustauschverbindung an den mindestens einen Rechner zu übermitteln, der dazu ausgebildet ist, anhand der Sensordaten zumindest eine der folgenden Informationen einem Bediener (insbesondere Arzt) des Katheters bereitzustellen: zumindest eine Instruktion zur Bedienung des Katheters, zumindest eine Handlungsempfehlung hinsichtlich eines mittels des Katheters durchzuführenden Eingriffs, zumindest ein unter Verwendung des Katheters gewonnenes Messergebnis.

Weiterhin ist gemäß einer Ausführungsform der Erfindung vorgesehen, dass das medizinische System den jeweiligen Katheter aufweist, an dem die Kommunikationseinrichtung lösbar, insbesondere klemmbar, festlegbar ist (siehe oben).

Im Folgenden sollen Ausführungsformen der Erfindung sowie weitere Merkmale und Vorteile der Erfindung anhand der Figuren erläutert werden. Es zeigen:
- Fig. 1: einen aus dem Stand der Technik bekannten Katheter,
- Fig. 2: eine Ausführungsform einer erfindungsgemäßen Kommunikationseinrichtung, und
- Fig. 3: eine Ausführungsform eines erfindungsgemäßen Systems mit einer Kommunikationseinrichtung nach Art der Figur 2 sowie einer Andockstation und einem Katheter.

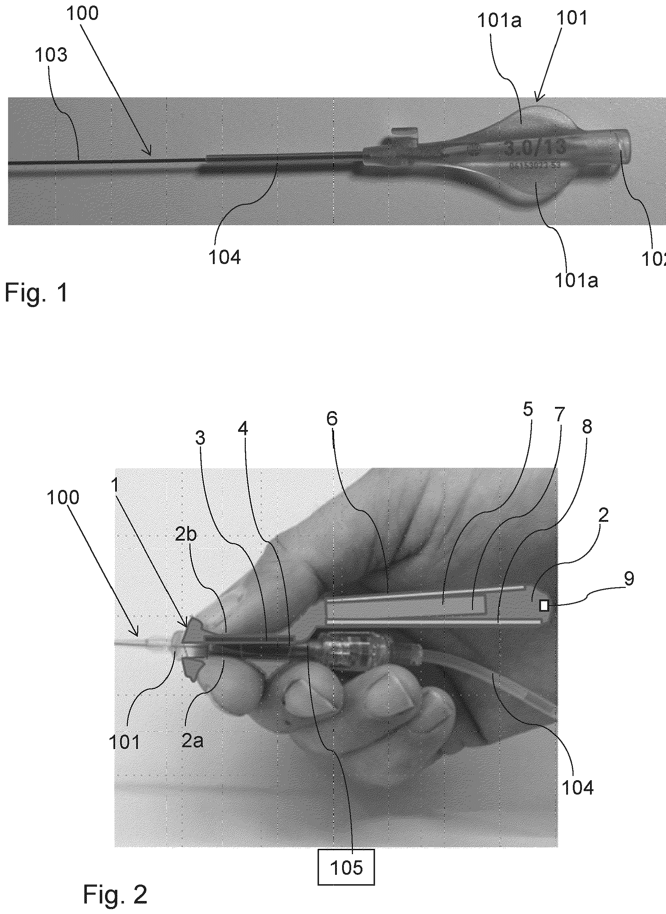

Figur 1 zeigt einen bekannten Katheter 100, mit einem proximalen Endabschnitt, der durch einen sogenannten Katheterhub 101 gebildet wird, der an einem proximalen Ende einen Anschluss 102 zum Einleiten eines fluiden Mediums (z.B. ein Inflationsmedium) in ein Lumen des Katheters 100 aufweist, wobei der Anschluss 102 vorzugsweise als Luer-Konnektor ausgebildet ist. Weiterhin weist der Katheterhub 101 zwei in entgegengesetzte Richtungen bzw. senkrecht zur axialen Richtung des Katheters 100 abstehende Flügel 101a auf, die für eine bessere Handhabbarkeit des Katheters 100 sorgen. Den Katheterhub 101, insbesondere dessen Flügel 101a, kann der Arzt bei der Einführung und Platzierung des Katheters 100 in der Hand halten. Weiterhin kann der Katheter 100 einen vom Katheterhub 101 abgehenden Stabilisator 104 aufweisen, der einen proximalen Abschnitt des Außenschaftes 103 zum Stabilisieren des Außenschaftes 103 umgibt.

Fig. 2 zeigt eine Ausführungsform einer erfindungsgemäßen Kommunikationseinrichtung 1 zur Nahfeldkommunikation N1 mit einem Katheter, z. B. mit einem Katheter 100 nach Art der Figur 1.

Die Kommunikationseinrichtung 1 weist eine NFC-Spule 3 und einem damit verbundenen Kommunikationsschaltkreis 4 auf, der für eine bidirektionale Nahfeldkommunikation N1 mit dem Katheter 10 konfiguriert ist. Der Katheter 100 kann hierzu einen entsprechenden Transponder 105 aufweisen, der über die NFC-Spule 3 zugleich mit Energie L1 versorgt wird. Weiterhin weist die Kommunikationseinrichtung 1 einen wiederaufladbaren Energiespeicher 5 und eine elektrische Ladespule 6 auf, die mit einem Ladeschaltkreis 7 zum Aufladen des Energiespeichers 5 verbunden ist. Bei der Ladespule 6 handelt es sich bevorzugt um eine NFC-Spule 6, so dass das Aufladen des Energiespeichers über eine induktive Kopplung vorgenommen werden kann. Die Kommunikationseinrichtung 1 weist ferner ein Gehäuse 2 auf, das die NFC-Spule 3, den Kommunikationsschaltkreis 4, den Energiespeicher 5, die Ladespule 6 und den Ladeschaltkreis 7 umgibt, wobei das Gehäuse 2 dazu ausgebildet ist zur Nahfeldkommunikation mit dem Katheter 10 lösbar an dem Katheter 10 festgelegt zu werden. Hierbei ist das Gehäuse 2 vorzugsweise als Griff ausgestaltet, der ergonomische Mulden 2a, 2b für den Zeigefinger und Daumen des Bedieners aufweisen kann (vergleiche Fig. 1). Im Gegensatz zur Fig. 1 zeigt Fig. 2 ebenfalls einen an den Luer-Konnektor 102 angeschlossene Leitung 104 zum Einleiten eines fluiden Mediums in das Lumen des Katheters 100.

Die Kommunikationseinrichtung 1 weist weiterhin bevorzugt eine Sende-Empfangseinheit 8 auf, die zur drahtlosen Kommunikation F der Kommunikationseinrichtung 1 mit einer weiteren Einrichtung (z.B. einer Andockstation 21, siehe unten) ausgebildet ist. Die Sende-Empfangseinheit 8 kann z.B. auf einem WLAN- oder Bluetooth-Standard beruhen oder anderen Übertragungsfrequenzen wie z.B. 433MHz / 898 MHz.

Weiterhin weist die Kommunikationseinrichtung 1 vorzugsweise eine optische Anzeige 9 zum Visualisieren eines Status der Kommunikationseinrichtung 1 auf. Die optische Anzeige 9 kann eine oder mehrere Leuchtquellen, wie z.B. eine oder mehrere LED, aufweisen.

Das Gehäuse 2 der Kommunikationseinrichtung 1 ist vorzugsweise so ausgestaltet, dass es die oben beschriebenen Komponenten hermetisch umschließt. Das Gehäuse 2 kann z.B. als Umspritzung der besagten Komponenten mit einem Kunststoff (z.B. POM) ausgebildet sein oder durch Vergießen der Komponenten mit einem Harz.

Hierbei ist bevorzugt die optische Anzeige 9 vollständig vom Gehäuse 2 der Kommunikationseinrichtung 1 umgeben, d.h., das Gehäuse 2 weist keine Durchgangsöffnung in einer Gehäusewandung des Gehäuses 2 für die optische Anzeige 9 aus. Das Gehäuse 2 kann für die optische Anzeige 9 einen die optische Anzeige 9 überdeckenden transluzenten oder transparenten Bereich aufweisen oder kann vollständig transluzent oder transparent ausgebildet sein.

Bevorzugt ist das Gehäuse 2 der Kommunikationseinrichtung 1 dazu ausgestaltet, mittels einer Klemmverbindung lösbar mit dem Katheter 100 bzw. mit dem Katheterhub 101 verbunden zu werden. Das Gehäuse 2 ist in dieser Ausgestaltung derart ausgeformt, dass es auf den Katheter 10, insbesondere auf den Katheterhub 101, geklemmt werden kann und somit lösbar mit dem Katheter 10 verbunden ist.

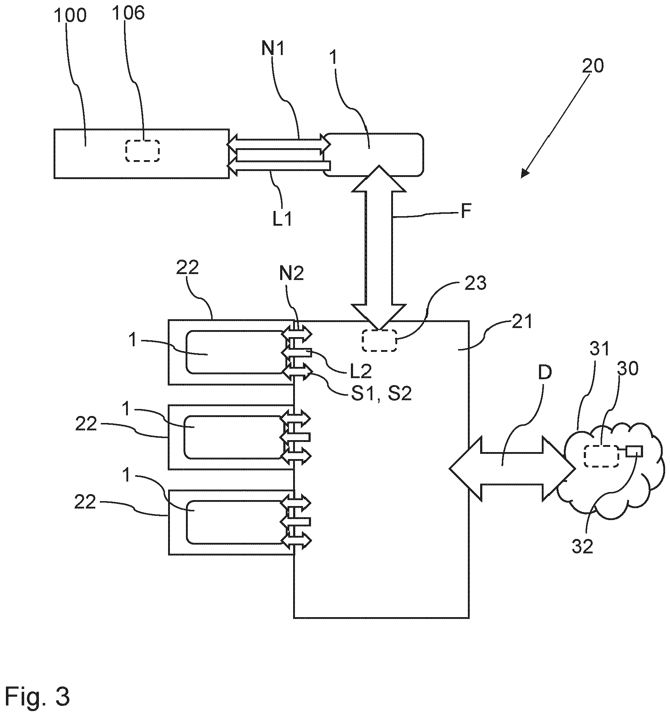

Figur 3 illustriert eine mögliche Einbettung einer erfindungsgemäßen Kommunikationseinrichtung 1 in ein medizinisches System 20, das auch den Katheter 100 umfassen kann, mit dem die Kommunikationseinrichtung 1 lösbar verbindbar ist.

Das medizinische System 20, weist weiterhin eine Andockstation 21 auf, wobei die mindestens eine Kommunikationseinrichtung 1 an die Andockstation 21 andockbar ist.

Gemäß einer Ausführungsform kann das System 20 mehrere erfindungsgemäße Kommunikationseinrichtungen 1 aufweisen, die jeweils an die Andockstation 21 andockbar sind, insbesondere gleichzeitig. Hierbei können die Kommunikationseinrichtungen 1 hinsichtlich des Katheters 100 bzw. hinsichtlich verschiedener Katheter auch unterschiedliche Funktionen aufweisen bzw. können nach verschiedenen, hierin beschriebenen Ausführungsformen ausgestaltet sein.

Die Andockstation 21 weist zum Andocken der mindestens einen bzw. der jeweiligen Kommunikationseinrichtung 1 vorzugsweise eine der Kommunikationseinrichtung 1 zugeordnete Aufnahme 22 zum Aufnehmen der Kommunikationseinrichtung 1 auf.

Bevorzugt ist die Andockstation 21 dazu ausgebildet, die jeweils angedockte bzw. in der entsprechenden Aufnahme 22 angeordnete Kommunikationseinrichtung 1 mit einer Lösung S1 zu Spülen und/oder die Kommunikationseinrichtung 1 durch Kontaktieren mit einem Sterilisationsmittel S2 zu sterilisieren. Die Lösung S1 bzw. das Sterilisationsmittel S2 können hierbei durch die Andockstation 21 in die jeweilige Aufnahme 22 eingeleitet und aus dieser wieder abgezogen werden.

Die Andockstation 21 ist weiterhin bevorzugt zum Laden des Energiespeichers 5 der jeweiligen Kommunikationseinrichtung 1 über die Ladespule 6 der Kommunikationseinrichtung 1 ausgebildet (L2), wenn die Kommunikationseinrichtung 1 an die Andockstation 21 angedockt ist. Hierbei kann die Andockstation 21 auch einen Systemtest der Kommunikationseinrichtung 1 über deren Ladespule 6 durchführen.

Weiterhin ist die Andockstation 21 bevorzugt dazu ausgebildet, die jeweilige Kommunikationseinrichtung 1 über die Ladespule 6 der jeweiligen Kommunikationseinrichtung 1 per Nahfeldkommunikation N2 zu konfigurieren, so dass insbesondere eine Kommunikationseinrichtung 1 nach dem Konfigurieren eine Funktion des Katheters 100, mit dem die Kommunikationseinrichtung 1 gepaart ist, über die NFC-Spule 3 der Kommunikationseinrichtung 1 steuern kann und/oder Daten aus dem Katheter 100 auslesen kann.

Zur drahtlosen Kommunikation der Andockstation 21 mit einer an den Katheter 100 lösbar festgelegten Kommunikationseinrichtung 1 weist die Andockstation 21 bevorzugt eine Sende-Empfangseinheit 23 auf, die zur drahtlosen Kommunikation F mit der Sende-Empfangseinheit 8 der Kommunikationseinrichtung 1 ausgebildet ist.

Weiterhin kann das medizinische System 20 zumindest einen Rechner 30 aufweisen, wobei die Andockstation 21 dazu ausgebildet ist, über eine Datenaustauschverbindung D an den mindestens einen Rechner 30 Daten zu übermitteln, die durch die mindestens eine Kommunikationseinrichtung 1 an die Andockstation 21 übermittelt worden sind (z.B. per Nahfeldkommunikation N1 im angedockten Zustand oder über die Sende-Empfangseinheiten 8, 23).

Gemäß einer Ausführungsform ist die mindestens eine Kommunikationseinrichtung 1 dazu ausgebildet, mittels ihrer NFC-Spule 3 und des Kommunikationsschaltkreises 4 katheterspezifische Daten aus dem Katheter 100 auszulesen und an die Andockstation 21 zu übermitteln (z.B. per Nahfeldkommunikation N1 im angedockten Zustand oder über die Sende-Empfangseinheiten 8, 23), wobei die Andockstation 21 vorzugsweise dazu ausgebildet ist, die katheterspezifischen Daten über die Datenaustauschverbindung D an den mindestens einen Rechner 30 zu übermitteln, der bevorzugt dazu ausgebildet ist, die katheterspezifischen Daten und/oder daraus erzeugte Daten auf einem Bildschirm 32 darzustellen. Der mindestens eine Rechner 30 kann Teil eines Rechnernetzwerks 31 sein, insbesondere ein Server 30 des Rechnernetzwerks 31. Bei dem Rechnernetzwerk 31 kann es sich um ein Rechnernetzwerk 31 eines Home Monitoring Service Centers (HMSC) handeln, wobei das Rechnernetzwerk 31 dazu ausgebildet sein kann, Patientendaten zu verwalten sowie insbesondere automatisch eine Verknüpfung von katheterspezifischen Daten mit Daten eines Eingriffes (unter Verwendung des Katheters 100) sowie mit Patientendaten des behandelten Patienten herzustellen, insbesondere zur Erzeugung einer Handlungsempfehlungen hinsichtlich eines zukünftigen Eingriffs an dem gleichen Patienten oder an einem anderen Patienten.

Die erfindungsgemäße Kommunikationseinrichtung 1 erlaubt insbesondere eine dezentrale Überwachung von Funktionen eines Katheters 100. So kann z.B. die Kommunikationseinrichtung gemäß einer Ausführungsform eine Medikamentenfreigabe durch den Katheter 100 (z.B. entsprechend ausgestatteter Drainagekatheter) initiieren und zeitgesteuert mittels des Katheters 100 durchführen. Die Funktion hierzu kann z.B. während des Sterilisations- und Ladevorganges des Energiespeichers 5 (siehe oben) konfiguriert werden, wobei ein Feedback zur Andockstation 21 hinsichtlich einer korrekten Durchführung der Medikamentenfreigabe möglich ist.

Weiterhin erlaubt eine erfindungsgemäße Kommunikationseinrichtung 1 mit Vorteil eine Bereitstellung funktionaler Kathetersensorik für ein übergeordnetes Analysesystem (z.B. in Form des mindestens einen Rechners 30 bzw. Rechnernetzwerks 31 und damit ggf. verbundener Komponenten). So kann z.B. die Kommunikationseinrichtung 1 dazu ausgebildet sein, eine resistive oder kapazitive Kathetersensorik mit dem übergeordneten Analysesystem zu verbinden und dieses mit entsprechenden Sensordaten zu versorgen.

Weiterhin erlaubt eine erfindungsgemäße Kommunikationseinrichtung 1 mit Vorteil das Auslesen katheterspezifischer Daten aus dem Katheter 100, wobei ein übergeordnetes System, z.B. der mindestens eine Rechner 30 bzw. das Rechnernetzwerk 31, detailliert sämtliche für den Kathetereingriff notwendigen Informationen auf einem Bildschirm 32 visualisiert. Die spezifisch erfassten Daten erlauben sowohl die Identifikation / Klassifikation des Katheters 100 wie auch eine zusätzliche Detailierung von Kathetereigenschaften durch z.B. katheterspezifische Kalibrierungsdaten.

Weiterhin können hierbei mittels erfassender Sensorik am Katheter 100 noch zusätzliche Eigenschaften, Instruktionen, Handlungsempfehlungen, und/oder Messergebnisse an den Benutzer weitergegeben werden.

Zusammenfassend weist die erfindungsgemäße Kommunikationseinrichtung 1 mit Vorteil die Befähigungen auf, ein medizinisches Gerät (z.B. einen Katheter 100) mit elektrischer Energie zu versorgen sowie eine Kommunikations- und Überwachungsfunktionalität für das medizintechnischen Gerät bereitzustellen. Das erfindungsgemäße Design unterstützt dabei eine vollständige Sterilisierbarkeit vor Ort, kann aber und auch die Möglichkeit einer zeitlich gesteuerte Überwachungs- oder Aktivierungsfunktion an diesem Kathetersystem unterstützen.

## Patentansprüche

1. Kommunikationseinrichtung (1) zur Nahfeldkommunikation mit einem Katheter (100), mit:
- einer NFC-Spule (3) und einem damit verbundenen Kommunikationsschaltkreis (4), der für eine bidirektionale Nahfeldkommunikation mit dem Katheter (100) konfiguriert ist,
- einem wiederaufladbaren Energiespeicher (5),
- einer elektrischen Ladespule (6), die mit einem Ladeschaltkreis (7) zum Aufladen des Energiespeichers (5) verbunden ist, und
- einem Gehäuse (2), das die NFC-Spule (3), den Kommunikationsschaltkreis (4), den Energiespeicher (5), die Ladespule (6) und den Ladeschaltkreis (7) umgibt, wobei das Gehäuse (2) dazu ausgebildet ist, zur Nahfeldkommunikation (N1) mit dem Katheter (100) lösbar an dem Katheter (100) festgelegt zu werden.

2. Kommunikationseinrichtung nach Anspruch 1, **dadurch gekennzeichnet, dass** das Gehäuse (2) als Griff ausgestaltet ist.

3. Kommunikationseinrichtung nach Anspruch 1 oder 2, **dadurch gekennzeichnet, dass** die Kommunikationseinrichtung (1) eine Sende-Empfangseinheit (8) aufweist, die zur drahtlosen Kommunikation mit einer Andockstation (21) konfiguriert ist.

4. Kommunikationseinrichtung nach einem der vorhergehenden Ansprüche, **dadurch gekennzeichnet, dass** die Kommunikationseinrichtung (1) eine optische Anzeige (9) aufweist, insbesondere in Form zumindest einer Leuchtdiode, wobei die Kommunikationseinrichtung (1) dazu konfiguriert ist, einen Zustand der Kommunikationseinrichtung (1) mittels der optischen Anzeige (9) anzuzeigen.

5. Kommunikationseinrichtung nach einem der vorhergehenden Ansprüche, **dadurch gekennzeichnet, dass** die Kommunikationseinrichtung (1) dazu ausgebildet ist, den Katheter (100) über die NFC-Spule (3) der Kommunikationseinrichtung (1) zur Abgabe eines Medikaments zu veranlassen.

6. Kommunikationseinrichtung nach einem der Ansprüche 1 bis 4, **dadurch gekennzeichnet, dass** der Katheter (100) zumindest einen Sensor (106) aufweist, insbesondere einen resistiven oder kapazitiven Sensor, wobei die Kommunikationseinrichtung (1) dazu ausgebildet ist, ein Ausgangssignal des mindestens einen Sensors (106) und/oder daraus ermittelte Daten mittels der NFC-Spule (3) und des Kommunikationsschaltkreises (4) auszulesen.

7. Kommunikationseinrichtung nach einem der vorhergehenden Ansprüche, **dadurch gekennzeichnet, dass** die Kommunikationseinrichtung (1) dazu konfiguriert ist, über die NFC-Spule (3) katheterspezifische Daten aus dem Katheter (100) auszulesen.

8. Medizinisches System (20), aufweisend zumindest eine Kommunikationseinrichtung (1) nach einem der vorhergehenden Ansprüche sowie eine Andockstation (21), wobei die mindestens eine Kommunikationseinrichtung (1) an die Andockstation (21) andockbar ist.

9. Medizinisches System nach Anspruch 8, **dadurch gekennzeichnet, dass** die Andockstation (21) zum Andocken der mindestens einen Kommunikationseinrichtung (1) eine Aufnahme (22) zum Aufnehmen der mindestens einen Kommunikationseinrichtung (1) aufweist.

10. Medizinisches System nach Anspruch 8 oder 9, **dadurch gekennzeichnet, dass** die Andockstation (21) dazu ausgebildet ist, die mindestens eine Kommunikationseinrichtung (1) mit einer Lösung (S1) zu Spülen und/oder die mindestens eine Kommunikationseinrichtung (1) durch Kontaktieren mit einem Sterilisationsmittel (S2) zu sterilisieren, wenn die mindestens eine Kommunikationseinrichtung (1) an die Andockstation (21) angedockt ist.

11. Medizinisches System nach einem der Ansprüche 8 bis 10, **dadurch gekennzeichnet, dass** die Andockstation (21) zum Laden (L2) des Energiespeichers (5) der mindestens einen Kommunikationseinrichtung (1) über die Ladespule (6) ausgebildet ist, wenn die mindestens eine Kommunikationseinrichtung (1) an die Andockstation (21) angedockt ist, und/oder dass die Andockstation (21) zur Durchführung eines Systemtests der mindestens einen Kommunikationseinrichtung (1) über die Ladespule (6) ausgebildet ist, wenn die mindestens eine Kommunikationseinrichtung (1) an die Andockstation (21) angedockt ist.

12. Medizinisches System nach eine der Ansprüche 8 bis 11, **dadurch gekennzeichnet, dass** die Andockstation (21) dazu ausgebildet ist, die mindestens eine Kommunikationseinrichtung (1) über die Ladespule (6) zu konfigurieren, so dass insbesondere die mindestens eine Kommunikationseinrichtung (1) nach dem Konfigurieren eine Funktion des Katheters (100) über die NFC-Spule (3) der mindestens einen Kommunikationseinrichtung (1) steuern kann und/oder Daten aus dem Katheter (100) auslesen kann.

13. Medizinisches System nach einem der Ansprüche 8 bis 12, **dadurch gekennzeichnet, dass** die Andockstation (21) eine Sende-Empfangseinheit (23) aufweist, die zur Kommunikation mit der mindestens einen Kommunikationseinrichtung (1) konfiguriert ist, insbesondere wenn die mindestens eine Kommunikationseinrichtung (1) bestimmungsgemäß lösbar an dem Katheter (100) festgelegt ist.

14. Medizinisches System nach einem der Ansprüche 8 bis 13, **dadurch gekennzeichnet, dass** das medizinische System (20) zumindest einen Rechner (30) aufweist, wobei die Andockstation (21) dazu ausgebildet ist, über eine Datenaustauschverbindung (D) an den mindestens einen Rechner (30) Daten zu übermitteln, die durch die mindestens eine Kommunikationseinrichtung (1) an die Andockstation (21) übermittelt worden sind.

15. Medizinisches System nach Anspruch 14, **dadurch gekennzeichnet, dass** die mindestens eine Kommunikationseinrichtung (1) dazu ausgebildet ist, mittels der NFC-Spule (3) und des Kommunikationsschaltkreises (4) katheterspezifische Daten aus dem Katheter (100) auszulesen und an die Andockstation (21) zu übermitteln, wobei die Andockstation (21) dazu ausgebildet ist, die katheterspezifischen Daten über die Datenaustauschverbindung (D) an den mindestens einen Rechner (30) zu übermitteln, der vorzugsweise dazu ausgebildet ist, die katheterspezifischen Daten und/oder daraus erzeugte Daten auf einem Bildschirm (32) darzustellen.
